# EUROPÄISCHE PATENTANMELDUNG

(11) **EP 2 799 023 A1**
(43) Veröffentlichungstag der Anmeldung: **05.11.2014**
(21) Anmeldenummer: 14156682.8
(22) Anmeldetag: 26.02.2014
(51) Int. Cl.: A61B 17/88, B25B 15/02

(54) **Schraubendreher für Knochenschrauben**

(30) Priorität: 29.04.2013 DE 202013004369 U
(71) Anmelder: Silony Medical International AG, 8500 Frauenfeld (CH)
(72) Erfinder: Heuer, Frank, 70794 Filderstadt (DE); Trautwein, Frank Thilo, 707094 Filderstadt (DE); Fromm, Michael, 72537 Mehrstetten (DE)
(74) Vertreter: DREISS Patentanwälte PartG mbB

(57) **Zusammenfassung**

Die Erfindung betrifft einen Schraubendreher (1) zum Betätigen von Knochenschrauben (2a, 2b) mit einem Schaft (3) und einem Gabelkopf (4a, 4b), welcher zur Halterung eines Wirbelsäulenstabes oder ähnlichem dient, mit einer zentralen Welle (10), welche an ihrem distalen Ende mit einem Form- oder Kraftschlussprofil (12) zum Eingriff in ein entsprechendes Gegenprofil an der Knochenschraube (2a, 2b) für ihre Drehung versehen ist, mit einer die Welle (10) drehbar übergreifenden distalen Hülse (20), welche mit Verbindungsmitteln (21) zur Halterung des Schraubendrehers (1) am Gabelkopf (4a, 4b) der Knochenschraube (2a, 2b) versehen ist, mit einem Haltegriff (50) am proximalen Ende des Schraubendrehers (1), und mit einem mit der Hülse (20) verbundenen Drehgriff (30), wobei eine Verstelleinrichtung (40) zur axialen Verstellung der relativen Lage der Welle (20) bezüglich der Hülse (10) in mindestens zwei unterschiedliche Positionen vorgesehen ist, welche für verschiedene Größen oder Formen von Knochenschrauben (2a, 2b) angepasst sind.

## Beschreibung

Die vorliegende Erfindung betrifft einen Schraubendreher zum Betätigen von Knochenschrauben nach dem Oberbegriff von Anspruch 1.

Solche Knochenschrauben werden an ihrer Bestimmungsstelle in einem Knochen, wie zum Beispiel einem Wirbelknochen im Körper eingeschraubt und dienen für eine Befestigung beispielsweise eines Wirbelsäulenstabes, der zwischen mehreren derartigen Schrauben an ihrem hierfür vorgesehenen Gabelkopf befestigt wird. Diese Knochenschrauben weisen in der Regel einen mit dem Gewinde versehenen Schaft sowie einen Gabelkopf in Form von gabelförmig vorragenden Schenkeln auf, zwischen welchen der Wirbelsäulenstab beispielsweise über Madenschrauben befestigt wird. Der Schraubenschaft ist drehbar im Verhältnis zum Gabelkopf gelagert und weist an seinem hinterem Ende ein Formschlussprofil oder Kraftschlussprofil auf, in welches ein Schraubendreher eingreifen kann, um die Schraube in den Knochen an der Bestimmungsstelle einzuschrauben oder herauszuschrauben. Hierzu sind herkömmliche derartige Schraubendreher mit einem Verbindungs- oder Haltemittel versehen, welches den Gabelkopf der Schraube beim Einschrauben des Schraubenschafts hält. Die Knochenschraube wird quasi am Gabelkopf durch den Schraubendreher gehalten, damit ein inneres Schraubenelement die Betätigung der Knochenschraube erlaubt.

Solche Knochenschrauben gibt es mit unterschiedlichen Längen von Gabelköpfen, um bei verschiedenen relativen Lagen von beispielsweise Wirbelknochen entsprechend passende Formen von Knochenschrauben einsetzen zu können. Das heißt, bei sogenannten Langkopfknochenschrauben sind die Schenkel des Gabelkopfs länger gegenüber sogenannten Kurzkopfknochen-schrauben. Um die beschriebene Betätigung solcher unterschiedlichen Typen von Knochenschrauben zu ermöglichen, wurden im Stand der Technik jeweils separate Schraubendreher eingesetzt, die auf den jeweiligen Schraubentyp entsprechend speziell angepasst waren. Dies hat den Nachteil, dass zum einen eine Vielzahl unterschiedlicher Schraubendreher für die verschiedenen Typen, Versionen oder Längen von Knochen-schrauben bereit gestellt werden müssen. Zum anderen ist die Handhabung dieser verschiedenen Knochenschrauben erschwert, da die jeweilige Zuordnung des passenden Schraubendrehers zu den entsprechenden Typen von Knochenschrauben von dem Nutzer korrekt vorgenommen werden muss, beispielsweise durch eine entsprechende Markierung oder Codierung.

Demgegenüber liegt der Erfindung die Aufgabe zugrunde, einen Schraubendreher zum Betätigen von Knochenschrauben bereit zu stellen, welcher für verschiedene Typen, Versionen und Längen von Knochenschrauben variabel einsetzbar ist. Außerdem soll mit der Erfindung die korrekte Verwendung von passenden Knochenschrauben für den jeweiligen Einsatzzweck erleichtert werden.

Diese Aufgabe wird erfindungsgemäß durch einen Schraubendreher mit den Merkmalen nach Anspruch 1 gelöst. Vorteilhafte Ausgestaltungen und Weiterbildungen der Erfindung sind Gegenstand der abhängigen Ansprüche.

Der Schraubendreher nach der Erfindung weist eine zentrale Welle auf, welche an ihrem distalen Ende mit einem Formoder Kraftschlussprofil zum Eingriff in ein entsprechendes Gegenprofil an der Knochenschraube für deren Drehung versehen ist, eine die Welle drehbar übergreifende distale Hülse, welche mit Verbindungsmitteln zur Halterung des Schrauben-drehers am Gabelkopf bzw. an der Knochenschraube versehen ist, einen Haltegriff am proximalen Ende des Schraubendrehers und einen mit der Hülse verbundenen Drehgriff, wobei der Schraubendreher dadurch gekennzeichnet ist, dass eine Verstelleinrichtung zur axialen Verstellung der relativen Lage der Welle bezüglich der Hülse in mindestens zwei unter-schiedliche Positionen vorgesehen ist, welche für verschiedene Größen oder Formen von Knochenschrauben angepasst sind. Der erfindungsgemäße Schraubendreher ist damit variabel einstellbar auf jeweilige unterschiedliche Formen oder Größen von Knochenschrauben. Mit der Verstelleinrichtung kann die relative Lage der zentralen Welle mit dem Form- oder Kraftschlussprofil bezüglich einer diese umgreifenden Hülse gezielt verstellt werden. Durch die mindestens zwei vordefinierten unterschiedlichen Positionen der Welle bezüglich der Hülse können mit ein und demselben Schraubendreher beispielsweise sogenannte Kurzkopfknochenschrauben ebenso wie Langkopfknochenschrauben betätigt werden. In den jeweiligen unterschiedlichen Positionen fixiert die Verstelleinrichtung die zentrale Welle drehbar innerhalb der distalen Hülse, so dass eine Halterung der Knochenschraube mit den Verbindungsmitteln der Hülse ermöglicht ist und dennoch die zentrale Welle ausreichend hervorragt, damit das Form- oder Kraftschlussprofil der Welle ausreichend vorsteht, um die Betätigung der Knochenschraube über einen Drehen des Schraubendrehers zu erlauben. Die Einsatzmöglichkeiten des Schraubendrehers sind damit vergrößert. Ein Wechsel zwischen unterschiedlichen Schrauben-drehern bei unterschiedlichen Typen von Knochenschrauben ist somit nicht mehr erforderlich.

Nach einer vorteilhaften Ausgestaltung der Erfindung weist die Verstelleinrichtung zum Einstellen von unterschiedlichen Positionen entsprechend unterschiedlicher Typen oder Größen von Knochenschrauben ein mechanisches Rastmittel auf, welches eine vordefinierte axiale Verstellung zwischen der zentralen Welle und der distalen Hülse des Schraubendrehers ermöglicht. Mittels einem mechanischen Rastmittel wird eine sichere Verstellung der axialen Position der Welle bezüglich der Hülse gewährleistet. Die Funktionssicherheit eines mechanischen Rastmittels ist hoch. Durch ein Lösen des mechanischen Rastmittels wird die Verstellung erlaubt und nach einem Einrasten ist eine sichere Festlegung der jeweiligen gewünschten vordefinierten Position gegeben.

Nach einer weiteren vorteilhaften Ausgestaltung der Erfindung ist die Verstelleinrichtung zur Verstellung der axialen relativen Lage im Drehgriff der distalen Hülse integriert. Auf diese Weise bleibt der Schraubendreher in seiner äußeren Form kompakt und es gibt keine störenden zusätzlichen, nach außen vorragenden Bauelemente durch die erfindungsgemäße Verstell-einrichtung. Außerdem wird hierdurch die Betätigung der Verstelleinrichtung erleichtert, da der Nutzer einfach an dem Drehgriff die Betätigung zur Verstellung direkt vornehmen kann.

Nach einer weiteren vorteilhaften Ausgestaltung des erfindungsgemäßen Schraubendrehers ist die zentrale Welle, welche mit dem zum Drehen der Schraube vorgesehenen Formoder Kraftschlussprofil versehen ist, mit mindestens zwei konzentrischen Querschnittsverjüngungen versehen, in welche ein federbelastetes Rastelement der Verstelleinrichtung eingreift. Durch die konzentrischen Querschnittsverjüngungen kann die relative Lage und damit die Position der Welle bezüglich der distalen Hülse exakt eingestellt werden und ein Drehen der Welle zum Betätigen der Knochenschraube ist dennoch gewährleistet. Die Verstelleinrichtung greift mit entsprechenden Gegenformen in die jeweiligen konzentrischen Querschnittsverjüngungen ein, so dass eine sichere Fixierung in der vorgesehenen Position gegeben ist.

Nach einer weiteren vorteilhaften Ausgestaltung der Erfindung ist die Verstelleinrichtung angepasst, eine gewisse axiale Verstellung bzw. Verschiebung der relativen Lage der zentralen Welle zu der distalen Hülse in jeder der mindestens zwei vordefinierten Positionen zu erlauben. Hiermit ist zwar der Abstand von mindestens zwei Positionen zueinander exakt entsprechend der gewünschten Längenverstellung definiert, jedoch lässt sich die Welle innerhalb dieser Positionen im Verhältnis zur Hülse für die Betätigung leicht etwas verschieben. Auf diese Weise wird trotz Fixierung mittels der Verstelleinrichtung das Einsetzen und Herausnehmen des Formschluss- bzw. Kraftschlussprofils in die Knochenschraube möglich. Dies erleichtert die Bedienung des Schraubendrehers.

Nach einer weiteren vorteilhaften Ausgestaltung der Erfindung weist die zentrale Welle eine Durchgangsöffnung auf. Die zentrale Welle ist also mit einer inneren Öffnung versehen, die durch die gesamte Länge der Welle hindurchgeht. Auf diese Weise kann in dem Schraubendreher ein Führungsdraht oder ähnliches eingesetzt werden, der sich bis zum hinteren Ende des Schraubendrehers erstreckt und beim Einschrauben der Knochenschraube als Positionierungsmittel verwendet werden kann. Beispielsweise kann so ein Führungsdraht durch den Schraubendreher und die Knochenschraube hindurchgeführt werden, der an der Bestimmungsstelle am Knochen vorab fixiert ist, damit die Knochenschraube an der richtigen Position eingeschraubt werden kann.

Nach einer weiteren vorteilhaften Ausgestaltung der Erfindung ist die distale Hülse des Schraubendrehers selbst in einer drehbaren Außenhülse aufgenommen. Die drehbare Außenhülse verhindert eine versehentliche Lockerung der Schraube beim Befestigen der Knochenschraube an ihrer Bestimmungsstelle. Ferner schützt die Außenhülse die darunter liegende distalen Hülsen und verhindert somit einen direkten Kontakt mit der Hand des Nutzers.

Nach einer weiteren vorteilhaften Ausgestaltung der Erfindung ist die Welle in Richtung zum distalen Ende des Schrauben-drehers hin federbelastet. Somit wird die zentrale Welle des Schraubendrehers mit einem leichten Druck in Richtung der zu betätigenden Knochenschraube beaufschlagt. Diese Vorspannung durch die Feder erleichtert die Bedienung des Schraubendrehers.

Nach einer weiteren vorteilhaften Ausgestaltung der Erfindung ist der Haltegriff am proximalen Ende des Schraubendrehers lösbar mit der zentralen Welle gekoppelt. Auf diese Weise können unterschiedliche Formen von Wellen an ein und demselben Haltegriff eingesetzt werden, beispielsweise mit unterschied-lichem Formschlussprofil für unterschiedliche Typen von Knochenschrauben. Auch ist so eine Reinigung der Einzelteile des Schraubendrehers erleichtert.

Nach einer weiteren vorteilhaften Ausgestaltung der Erfindung entspricht der Abstand der mindestens zwei vordefinierten unterschiedlichen Positionen der relativen Lage zwischen der Welle und der Hülse einem standardisierten Längenunterschied von unterschiedlich langen Gabelköpfen der Knochenschrauben. Somit können standardisierte Langkopfknochenschrauben ebenso wie Kurzkopfknochenschrauben mit ein und demselben Schraubendreher ohne Probleme betätigt werden.

Nach einer weiteren vorteilhaften Ausgestaltung des erfindungsgemäßen Schraubendrehers ist die Verstelleinrichtung ein senkrecht zur Längsrichtung des Schraubendrehers wirkender, federbelasteter Drucktaster, welcher eine die Welle aufnehmende Ausnehmung und eine mit dem konzentrischen Querschnittsverjüngungen zusammenwirkende Austragung in der Ausnehmung derart aufweist, dass bei Betätigung des Druck-tasters eine Verstellung der Welle zur Hülse ermöglicht wird und bei Nichtbetätigung des Drucktasters eine Verstellung verhindert wird. Damit ist die Verstelleinrichtung sehr kompakt. Zudem ist eine hohe Funktionssicherheit der Verstelleinrichtung aufgrund des federbelasteten Drucktasters mit der mittigen Ausnehmung für die Aufnahme der Welle gegeben.

Nach einer weiteren vorteilhaften Ausgestaltung der Erfindung weist die distale Hülse an ihrem proximalen Ende ein Gewinde auf, welches zur Befestigung in einem Gegengewinde an der Knochenschraube dient. Die Knochenschrauben weisen in der Regel an ihrem Gabelkopf ein Innengewinde auf, das zum späteren Einschrauben einer Madenschraube zur Befestigung beispielsweise eines Wirbelsäulenstabes dient. In dieses Gewinde kann die Hülse für die Halterung des Gabelkopfes eingeschraubt werden und nach dem Befestigen der Knochenschraube leicht wieder herausgeschraubt werden.

Nach einer weiteren vorteilhaften Ausgestaltung des erfindungsgemäßen Schraubendrehers ist der Haltegriff als ein quer zur Längsrichtung der Welle verlaufender, im Wesentlichen T-förmiger Handgriff am proximalen Ende des Schraubendrehers. Eine einfache Bedienung des Schraubendrehers ist dadurch gewährleistet und es kann auch ein ausreichender Druck durch den Nutzer leicht aufgebracht werden. Außerdem wird hierdurch ein sicherer Halt des Schraubendrehers mit den beiden Händen des Nutzers gewährleistet.

Nach einer weiteren vorteilhaften Ausgestaltung der Erfindung ist der Drehgriff als ein sich in Längsrichtung der Welle erstreckender hohler Werkzeuggriff ausgebildet. Im inneren Hohlraum des Drehgriffs kann somit die Verstelleinrichtung gemäß der Erfindung leicht integriert werden. Die Handhabung des Schraubendrehers ist außerdem hierdurch erleichtert, da der Nutzer mit einer Hand am Drehgriff und mit der anderen Hand an dem vorderen Haltegriff den Schraubendreher sicher halten kann während dem Einschrauben oder Herausschrauben der Knochenschrauben. Nach einer weiteren vorteilhaften Ausgestaltung der Erfindung ist im Inneren der Durchgangsöffnung der Welle ein Führungsdraht vorgesehen, welcher zur Festlegung und zum Auffinden der korrekten Position der Bestimmungsstelle der Knochenschraube im Knochen dient. Der Führungsdraht kann ohne Störung der Funktion des Schraubendrehers durch das Innere der Welle vollständig hindurchgeführt werden und nach dem Anbringen der Knochenschraube leicht wieder entfernt werden. Solch ein Führungsdraht wird üblicherweise an dem Knochen vorab befestigt, damit die darauf aufgefädelte Knochenschraube mit dem Schraubendreher an der richtigen Stelle am Knochen eingeschraubt werden kann.

Nach einer weiteren vorteilhaften Ausgestaltung der Erfindung ist/sind das Form- oder Kraftschlussprofil des Schrauben-drehers und/oder das Verbindungsmittel an der distalen Hülse austauschbar ausgebildet. Die Einsatzvielfalt des Schraubendrehers wird hierdurch erhöht. Unterschiedliche Formen, Größen oder Arten von Knochenschrauben können mit ein und demselben Schraubendreher durch Austausch der entsprechen-den Teile betätigt werden.

Weitere Merkmale, Vorteile und Ausgestaltungen des erfindungs-gemäßen Schraubendrehers werden im Folgenden mehr im Detail anhand von einem Ausführungsbeispiel unter Bezugnahme auf die beigefügten Zeichnungen beschrieben werden. In den Zeichnungen zeigen:
Fig. 1a eine Seitenansicht eines Ausführungsbeispiels eines erfindungsgemäßen Schraubendrehers in einer Betriebsstellung für den Einsatz bei Knochenschrauben mit kurzem Gabelkopf;
Fig. 1b eine Seitenansicht des in der Fig. 1a gezeigten Ausführungsbeispiels eines erfindungsgemäßen Schraubendrehers in einer Betriebsposition für den Einsatz bei Knochenschrauben mit langem Gabelkopf;
Fig. 2 eine perspektivische Darstellung des Ausführungsbeispiels eines erfindungsgemäßen Schraubendrehers;
Fig. 3 eine perspektivische Explosionsansicht des Ausführungsbeispiels eines erfindungsgemäßen Schraubendrehers;
Fig. 4a eine Seitenansicht des Ausführungsbeispiels des erfindungsgemäßen Schraubendrehers;
Fig. 4b einen Längsschnitt des Ausführungsbeispiels des erfindungsgemäßen Schraubendrehers gemäß A-A aus der Fig. 4a;
Fig. 4c eine weitere Seitenansicht des
Ausführungsbeispiels eines erfindungsgemäßen Schraubendrehers; und
Fig. 4d eine Querschnittsansicht des Ausführungsbeispiels des erfindungsgemäßen Schraubendrehers gemäß B-B aus der Fig. 4c.

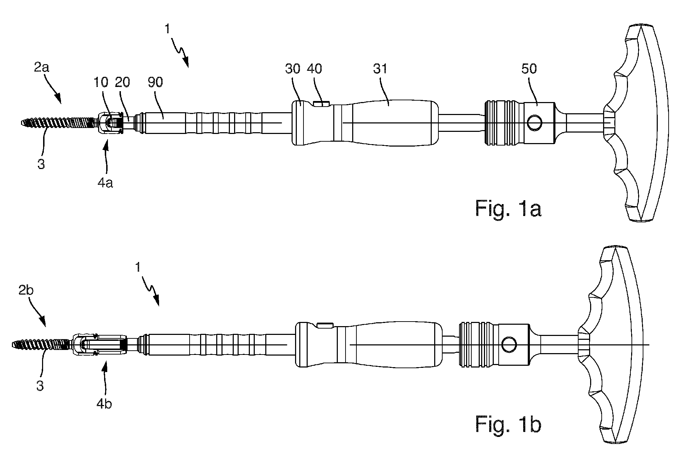

Die Figuren 1a und 1b zeigen in jeweiligen Seitenansichten ein Ausführungsbeispiel eines erfindungsgemäßen Schraubendrehers 1 für Knochenschrauben 2a, 2b. Die Fig. 1a zeigt das Ausführungsbeispiel des erfindungsgemäßen Schraubendrehers 1 in einer Betriebsstellung für den Einsatz bei Knochenschrauben 2a mit einem kurzen Gabelkopf 4a, das heißt einem Gabelkopf, dessen seitliche vorragende Schenkel vergleichsweise kurz ausgeführt sind. Die Fig. 1b zeigt den Schraubendreher 1 in einer Betriebsposition für den Einsatz bei einer anderen Art von Knochenschrauben 2b, nämlich Knochenschrauben 2b, welche einen langen Gabelkopf 4b aufweisen. Die Knochenschrauben 2a, 2b weisen einen Schaft 3 und einen mit diesem Schaft drehbar verbundenen Gabelkopf 4a, 4b auf. Das vordere Ende des Schraubendrehers 1 ist mit einem Formschlussprofil an der Welle 10 versehen, beispielsweise einem Torx-Profil, welches zum Drehen des Schraubenschaftes 3 dient. Um unterschiedliche Typen von Knochenschrauben 2a, 2b mit ein und demselben Schraubendreher 1 gemäß der Erfindung betätigen zu können, ist der Schraubendreher 1 mit einer Verstelleinrichtung 40 zur Verstellung der relativen Lage zwischen der zentralen Welle 10 und einer äußeren Hülse 20 versehen. Die Verstelleinrichtung 40 ist hier in Form eines Drucktasters in einem Drehgriff 30 realisiert, durch dessen Betätigung die zentrale Welle 10 von ihrer in der Fig. 1a dargestellten Position für kurze Knochenschrauben 2a in die demgegenüber ausgefahrene Position der Fig. 1b verstellt werden kann, welche für Knochenschrauben 2b mit einem längeren Gabelkopf 4b angepasst ist. Der Schraubendreher 1 weist an seinem proximalen Ende einen Haltegriff 50 auf, welcher zum Halten mit einer Hand des Bedieners dient, während die andere Hand den Schraubendreher an dem Drehgriff 30 hält. Die innere Welle 10 ist über den Haltegriff 50 drehbar, während die distale Hülse 20 über das Gewinde an dem Gabelkopf 4a, 4b befestigt ist (vgl. Fig. 1a und Fig. 1b). An der Außenseite der distalen Hülse 20 ist eine drehbare Außenhülse 90 vorgesehen, so dass ein unbeabsichtig-tes Lösen verhindert wird. Die spezielle Form und Ausgestal-tung der Verstelleinrichtung 40 wird im Nachfolgenden mehr im Detail unter Bezugnahme auf die weiteren Figuren dieser Ausführungsform erläutert werden.

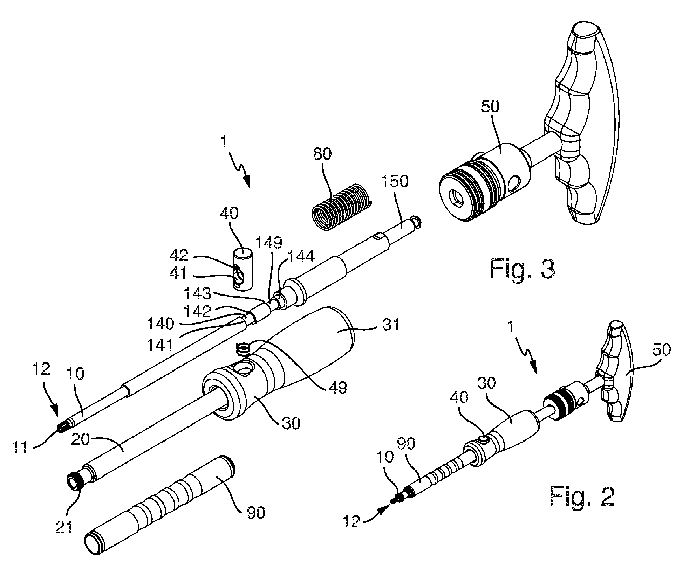

Das Ausführungsbeispiel des erfindungsgemäßen Schraubendrehers ist in der Fig. 2 in einer perspektivischen Ansicht und in der Fig. 3 in einer perspektivischen Explosionsansicht gezeigt. Der Schraubendreher 1 für Knochenschrauben gemäß der Erfindung weist bei diesem Ausführungsbeispiel eine zentrale Welle 10 auf, die an ihrem distalen Ende ein Formschlussprofil 12 aufweist, welches zum Eingriff in ein entsprechendes Gegenprofil an der Knochenschraube dient. Das Formschlussprofil 12 kann beispielsweise eine Torx-Kontur sein oder ein anderes Profil, welches bei derartigen Knochenschrauben vorhanden ist, und welches zur Drehmomentübertragung auf die Knochenschraube dient. An dem proximalen Ende ist die zentrale Welle 10 mit einem Verbindungszapfen 150 versehen, der zur lösbaren Befestigung am Haltegriff 50 dient. Die Welle 10 ist im Inneren einer distalen Hülse 20 und eines Drehgriffs 30 drehbar aufgenommen. Die Hülse 20 ist an ihrem freien Ende mit einem Gewinde 21 versehen für eine Verbindung mit einem entsprechenden Innengewinde an dem Gabelkopf der zu betätigenden Knochenschraube (vgl. Fig. 1a und Fig. 1b). In dem Drehgriff 30 ist eine erfindungsgemäße Verstelleinrichtung 40 in Form eines Drucktasters realisiert, mittels welcher eine axiale Lage zwischen der Welle 10 und der Hülse 20 verstellt werden kann. Die Verstelleinrichtung 40 besteht aus einem federbelastenden Drucktaster mit einer Ausnehmung 42, durch welche die Welle 10 im zusammengebauten Zustand hindurch-geführt ist. In der Ausnehmung 42 ist eine Austragung 41 vorgesehen, die mit zwei konzentrischen Querschnitts-verjüngungen 140, 149 der Welle 10 zusammenwirkt, um die vordefinierten Positionen bei der Verstellung durch die Verstelleinrichtung 40 einzustellen. Bei Betätigung des Drucktasters 40 lässt sich die Welle 10 von der einen konzentrischen Querschnittsverjüngung 140 zu der anderen konzentrischen Querschnittsverjüngung 149 verstellen und zurück. Auf diese Weise sind die verschiedenen Einstellungen des Schraubendrehers 1 für einerseits die Knochenschrauben 2a mit kurzem Gabelkopf 4a und andererseits die Knochenschrauben 2b mit langem Gabelkopf 4b ermöglicht, die in den zuvorigen Figuren 1a und 1b gezeigt sind.

Die distale Hülse 20 ist in einer drehbaren Außenhülse 90 aufgenommen, so dass eine unbeabsichtigte Lockerung der Knochenschraube vermieden wird, wenn diese mit dem Schraubendreher 1 gedreht wird. Die zum Schrauben der Knochenschraube dienende Welle 10 ist bei diesem Ausführungs-beispiel mit einer Druckfeder 80 vorgespannt, so dass eine leichtere Bedienung des Schraubendrehers 1 gegeben ist. Die Welle 10 ist hierfür mit einem kragenartigen Vorsprung versehen, gegen den die Druckfeder 80 im Inneren des Drehgriffs 30 zur Anlage kommt, so dass immer eine Vorspannung der Welle 10 in Richtung zu der zu betätigenden Knochen-schraube gegeben ist.

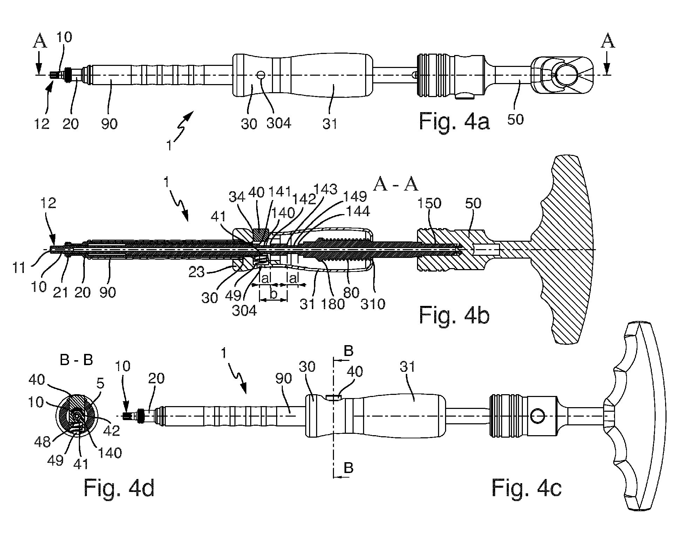

Die Figuren 4a, 4b, 4c und 4d zeigen jeweilige Seitenansichten bzw. Schnittansichten des Ausführungsbeispiels eines erfindungsgemäßen Schraubendrehers 1, anhand welcher die Verstelleinrichtung 40 im Folgenden mehr im Detail beschrieben wird. Wie man es insbesondere aus der Längsschnittansicht der Fig. 4b erkennen kann, weist der Schraubendreher 1 eine zentrale Welle 10 auf, die im Inneren einer distalen Hülse 20 und eines Drehgriffs 30 gelagert ist. Die Welle 10 mit dem Haltegriff 50 über einen Verbindungszapfen 150 drehfest gekoppelt, so dass der Haltegriff 50 von der Welle 10 bei Bedarf abgenommen werden kann. Die Welle 10 weist an ihrem distalen Ende ein Formschlussprofil 12 auf, das beispielsweise eine Torx-Kontur oder ein anderes für die Betätigung von Schrauben geeignetes Profil sein kann. An dem vorderen Ende ist die Welle 10 in einer distalen Hülse 20 drehbar aufgenommen, die fest mit dem Drehgriff 30 verbunden ist. Die distale Hülse 20 weist ein Gewinde 21 auf, das zur Befestigung für die Halterung der Knochenschraube beim Drehen der Schraube dient. Die distale Hülse 20 selbst ist in einer Außenhülse 90 aufgenommen, so dass ein unbeabsichtigtes Lösen der Schraube beim Drehen verhindert wird. Der Drehgriff 30 ist hohl ausgebildet, so dass in seinem Inneren einerseits die Welle 10 drehbar hindurchgeführt werden kann und andererseits die erfindungsgemäße Verstelleinrichtung 40 im Drehgriff 30 aufgenommen werden kann.

Bei diesem Ausführungsbeispiel ist die Verstelleinrichtung des Schraubendrehers 1 in Form eines federbelastenden Drucktasters 40 vorgesehen, der in dem Drehgriff 30 integriert ist. Der Drucktaster 40 ist im Wesentlichen quer zur Längsrichtung des Schraubendrehers 1 in einer dafür vorgesehenen Öffnung im Drehgriff 30 eingesetzt. Der Drucktaster 40 ist mit einer Ausnehmung 42 versehen, durch welche die Welle 10 hindurchgeführt ist. In der Ausnehmung 42 des Drucktasters 40 ist eine Austragung 41 vorhanden, die insbesondere im Vergleich zwischen der Längsschnittansicht der Fig. 4b und der Querschnittsansicht der Fig. 4d zu erkennen ist. Die Austragung 41 ist auf die Form von zwei konzentrischen Querschnittsverjüngungen 140, 149 der Welle 10 angepasst. Wenn der Drucktaster 40 nicht betätigt ist (Position der Figuren 4b und 4d), ist die Querschnittsverjüngung 140 in Eingriff mit der Austragung 41, so dass die relative axiale Lage zwischen der Welle 10 und der Hülse 20 festgelegt ist. Wenn der Drucktaster 40 gegen die Kraft der Feder 49 betätigt wird, lässt sich die Welle 10 in der axialen Richtung verstellen. Der Verstellweg zwischen den beiden Querschnittsverjüngungen 140, 149 ist in der Fig. 4b mit "b" eingezeichnet. Dieser Verstellweg "b" entspricht einem Unterschied in der Länge des Gabelkopfes 4a, 4b von verschieden großen Knochenschrauben 2a, 2b, wie sie in den Figuren 1a und 1b gezeigt sind. Jede der Querschnittsverjüngungen 140, 149 ist mit hinteren und vorderen Begrenzungsflächen 141, 142, 143, 144 versehen, gegen die die Austragung 41 des Drucktasters 40 anstößt. Die Welle 10 kann bezüglich der Hülse 20 bzw. dem Drehgriff 30 damit innerhalb der jeweiligen vordefinierten Position etwas verstellt werden, was mit den Abständen "a" in der Fig. 4b gezeigt ist. Auf diese Weise ist eine gewisse Verschiebung innerhalb der Begrenzungsflächen 141, 142 bzw. 143, 144 möglich, während trotzdem die jeweils ausgewählte vordefinierte Position durch die Verstelleinrichtung 40 eingehalten wird. Im Inneren des Drehgriffs 30 ist ferner eine Druckfeder 80 aufgenommen, welche gegen eine Abstützfläche 180 und eine hintere Wand 310 des Drehgriffs anliegt, so dass die Welle 10 mit einem leichten Druck in Richtung des distalen Endes des Schraubendrehers 1 vorgespannt ist. Diese Vorspannung erleichtert die Bedienung des Schraubendrehers 1. Auf Höhe des Drucktasters 40 ist an einer gegenüberliegenden Seite der Drehgriff 30 mit einer Öffnung 304 versehen, durch welche eine Reinigung der innen liegenden Bauelemente erleichtert wird.

Wie es insbesondere in der Querschnittsansicht der Fig. 4d zu erkennen ist, ist der Drucktaster der Verstelleinrichtung 40 bei diesem Ausführungsbeispiel mit einer mittigen in etwa kreisförmigen Ausnehmung 42 versehen, die ein Hindurchgehen und Verschieben der Welle 10 auch außerhalb der Querschnitts-verjüngung 140 erlaubt. Der Drucktaster 40 weist einen Hohlraum 48 auf, in welchem eine Druckfeder 49 aufgenommen wird, durch welchen der Drucktaster 40 vorgespannt ist. In der vorgespannten Position liegt die Querschnittsverjüngung 140 der Welle 10 innerhalb der Austragung 41, die auf den geringeren Durchmesser der Querschnittsverjüngung 140 angepasst ist, so dass die Welle 10 sicher in ihrer axialen Lage fixiert ist.

Mit dem erfindungsgemäßen Schraubendreher können unterschied-liche Größen oder Typen von Knochenschrauben 2a, 2b mit ein und demselben Schraubendreher gedreht werden. Durch einfaches Verstellen der Verstelleinrichtung 40 von einer vordefinierten Position in eine andere vordefinierte Position lassen sich die unterschiedlichen Typen oder Größen von Knochenschrauben betätigen. Der erfindungsgemäße Schraubendreher ist relativ einfach aufgebaut und weist eine kompakte Bauform auf. Die mechanische Verstelleinrichtung 40 ist vollständig im Inneren des Drehgriffs integriert. Durch die Möglichkeit einer Verstellung mittels der Verstelleinrichtung 40 sind die Einsatzmöglichkeiten und die Variabilität des Schraubendrehers 1 erhöht.

Die vorliegende Erfindung ist nicht auf die dargestellten Ausführungsformen beschränkt und es können verschiedene Änderungen und Modifikationen im Umfang der beigefügten Ansprüche vorgenommen werden: Beispielsweise können mehr als zwei vordefinierte Positionen der relativen axialen Lage der Welle 10 zu dem Drehgriff 30 bzw. der Hülse 20 vorgesehen sein, so dass eine noch größere Anzahl von unterschiedlichen Typen von Knochenschrauben mit dem gleichen Schraubendreher 1 gedreht werden können.

Außerdem kann anstatt der beschriebenen mechanischen Verstelleinrichtung 40 eine elektrische oder andersartig ausgestaltete Verstelleinrichtung vorgesehen sein. Die Verstelleinrichtung ist auch nicht auf einen federbelastenden Drucktaster beschränkt und es können andere Mittel für die axiale Verstellung der Welle in die verschiedenen Positionen vorgesehen sein, wie sie dem Fachmann des Gebiets bekannt sind. Auch ist der erfindungsgemäße Schraubendreher nicht auf die dargestellte Ausgestaltung des Haltegriffs 50 und des Drehgriffs 30 beschränkt. Anstatt eines im Wesentlichen T-förmigen Haltegriffs 50 kann beispielsweise auch ein konventioneller axialer Schraubendrehergriff vorgesehen sein. Die Anbindung des Haltegriffs 50 an der Welle 10 kann lösbar ausgestaltet sein, jedoch kann ebenso eine feste Verbindung zwischen der Welle 10 und dem Haltegriff 50 vorgesehen werden.

## Patentansprüche

1. Schraubendreher (1) zum Betätigen von Knochenschrauben (2a, 2b) mit einem Schaft (3) und einem Gabelkopf (4a, 4b), welcher zur Halterung eines Wirbelsäulenstabes oder ähnlichem dient, mit einer zentralen Welle (10), welche an ihrem distalen Ende mit einem Form- oder Kraftschlussprofil (12) zum Eingriff in ein entsprechendes Gegenprofil an der Knochenschraube (2a, 2b) für ihre Drehung versehen ist, mit einer die Welle (10) drehbar übergreifenden distalen Hülse (20), welche mit Verbindungsmitteln (21) zur Halterung des Schraubendrehers (1) am Gabelkopf (4a, 4b) der Knochenschraube (2a, 2b) versehen ist, mit einem Haltegriff (50) am proximalen Ende des Schraubendrehers (1), und mit einem mit der Hülse (20) verbundenen Drehgriff (30), **dadurch gekennzeichnet, dass** eine Verstelleinrichtung (40) zur axialen Verstellung der relativen Lage der Welle (20) bezüglich der Hülse (10) in mindestens zwei unterschiedliche Positionen vorgesehen ist, welche für verschiedene Größen oder Formen von Knochenschrauben (2a, 2b) angepasst sind.

2. Schraubendreher nach Anspruch 1, **dadurch gekennzeichnet, dass** die Verstelleinrichtung (40) ein mechanisches Rastmittel aufweist, welches eine vordefinierte axiale Verstellung zwischen der Welle (20) und der Hülse (10) ermöglicht.

3. Schraubendreher nach Anspruch 1 oder 2, **dadurch gekennzeichnet, dass** die Verstelleinrichtung (40) im Drehgriff (30) der distalen Hülse (20) integriert ist.

4. Schraubendreher (1) nach einem der vorhergehenden Ansprüche, **dadurch gekennzeichnet, dass** die zentrale Welle (10) mit mindestens zwei konzentrischen Querschnittsverjüngungen (140, 149) versehen ist, in welche ein federbelastetes Rastelement der Verstelleinrichtung (40) eingreift.

5. Schraubendreher nach einem der vorhergehenden Ansprüche, **dadurch gekennzeichnet, dass** die Verstelleinrichtung (40, 140, 149) angepasst ist, eine gewisse axiale Verstellung der relativen Lage der zentralen Welle (20) zu der distalen Hülse (10) in jeder der vordefinierten Positionen zu erlauben.

6. Schraubendreher nach einem der vorhergehenden Ansprüche, **dadurch gekennzeichnet, dass** die zentrale Welle (20) eine Durchgangsöffnung (11) aufweist.

7. Schraubendreher nach einem der vorhergehenden Ansprüche, **dadurch gekennzeichnet, dass** die distale Hülse (10) in einer drehbaren Außenhülse (90) aufgenommen ist.

8. Schraubendreher nach einem der vorhergehenden Ansprüche, **dadurch gekennzeichnet, dass** die Welle (20) in Richtung zum distalen Ende hin federbelastet ist.

9. Schraubendreher nach einem der vorhergehenden Ansprüche, **dadurch gekennzeichnet, dass** der Haltegriff (50) lösbar mit der zentralen Welle (20) gekoppelt ist oder als ein quer zur Längsrichtung der Welle (10) verlaufender T-förmiger Handgriff am proximalen Ende des Schraubendrehers (1) ausgebildet ist.

10. Schraubendreher nach einem der vorhergehenden Ansprüche, **dadurch gekennzeichnet, dass** der Abstand der mindestens zwei vordefinierten unterschiedlichen Positionen der relativen Lage zwischen der Welle (20) und der Hülse (10) einem standardisierten Längenunterschied von Gabelköpfen (4a, 4b) der Knochenschrauben (2a, 2b) entspricht.

11. Schraubendreher nach einem der vorhergehenden Ansprüche, **dadurch gekennzeichnet, dass** die Verstelleinrichtung (40) ein senkrecht zur Längsrichtung des Schraubendrehers (1) wirkender, federbelasteter Drucktaster (40) ist, welcher eine die Welle aufnehmende Ausnehmung (42) und eine mit den konzentrischen Querschnittsverjüngungen (140, 149) zusammenwirkende Austragung (41) in der Ausnehmung derart aufweist, dass bei Betätigung des Drucktasters (40) eine Verstellung der Welle (10) zur Hülse (20) ermöglicht wird und bei Nichtbetätigung verhindert wird.

12. Schraubendreher nach einem der vorhergehenden Ansprüche, **dadurch gekennzeichnet, dass** die distale Hülse (20) an ihrem proximalen Ende ein Gewinde (21) aufweist, welches zur Befestigung in einem Gegengewinde an der Knochenschraube (2a, 2b) dient.

13. Schraubendreher nach einem der vorhergehenden Ansprüche, **dadurch gekennzeichnet, dass** der Drehgriff (30) als ein sich in Längsrichtung der Welle (20) ersteckender Werkzeuggriff mit einem inneren Hohlraum ausgebildet ist.

14. Schraubendreher nach einem der vorhergehenden Ansprüche, **dadurch gekennzeichnet, dass** im Innern der Durchgangsöffnung (11) der Welle (20) ein Führungsdraht vorgesehen ist, welcher zur Festlegung und zum Auffinden der Position der Bestimmungsstelle der Knochenschraube (2a, 2b) im Knochen dient.

15. Schraubendreher nach einem der vorhergehenden Ansprüche, **dadurch gekennzeichnet, dass** das Form- oder Kraftschlussprofil (12) des Schraubendrehers (1) und/oder das Verbindungsmittel (21) an der Hülse (20) austauschbar ausgebildet ist/sind.
